# EUROPÄISCHE PATENTSCHRIFT

(11) **EP 2 476 372 B1**
(45) Veröffentlichungstag und Bekanntmachung des Hinweises auf die Patenterteilung: **14.05.2014**
(21) Anmeldenummer: 12150815.4
(22) Anmeldetag: 11.01.2012
(51) Int. Cl.: A61B 5/107, B68F 3/00, G01B 5/20, G01B 3/04, G01B 3/10

(54) **Vorrichtung zur dreidimensionalen Abbildung von Pferderücken**
Device for three-dimensional imaging of horse backs
Dispositif de représentation tridimensionnelle de dos de chevaux

(30) Priorität: 18.01.2011 DE 102011002829; 02.09.2011 DE 102011053233
(43) Veröffentlichungstag der Anmeldung: 18.07.2012
(73) Patentinhaber: Büttner, Thomas, 01169 Dresden (DE)
(72) Erfinder: Büttner, Thomas, 01169 Dresden (DE)
(74) Vertreter: Sperling, Thomas

(56) Entgegenhaltungen:
- WO-A1-97/17281
- WO-A1-2010/103108
- GB-A- 1 587 688
- US-A1- 2003 221 328
- US-A1- 2004 112 017
- YOUTUBE (SATTLEREIBUETTNER): "Vermessen des Pferderückens / Einstellung TOMAX", 20110708, 8. Juli 2011 (2011-07-08), Seiten 1-17, XP007920576,
- FINDUS REITSPORT: "Bundeseinheitliches System zur Vermessung des Pferderückens", 20100611, 11. Juni 2010 (2010-06-11), Seiten 1-3, XP007920575,
- ANONYMOUS: "Sattelmassblatt des BVFR", 20100000, 2010, Seiten 1-2, XP007920574,

## Beschreibung

Die Erfindung betrifft eine Vorrichtung zur dreidimensionalen maßstabsgerechten Abbildung von Pferderücken.

Die anatomisch naturgetreue Darstellung des Pferderückens im dreidimensionalen Raum ist beispielsweise erforderlich, um Pferdesättel anzufertigen, die präzise an die Anatomie jeweiligen Pferdes angepasst sind.

Wird ein Sattel nicht spezifisch für ein bestimmtes Tier angefertigt, so ergeben sich Probleme beim Gebrauch von nicht exakt passenden Sätteln durch Verspannungen der Muskulatur und Störung des Bewegungsablaufes des Pferdes. Diesen Problemen wird teilweise dadurch entgegengewirkt, dass nach der DE 600 12 949 T2 mittels Abdruckkissen zum Sichtbarmachen eines Abdruckmusters der Druckverteilung eines Sattels spezielle Unterlagen für die nicht exakt passenden Sättel entwickelt werden.

Bei der Neuanfertigung von Reitsätteln ist es wünschenswert, eine größtmögliche Präzision und Passform zu erreichen, um nicht zusätzliche Aufwendungen durch Anpassungen des Sattels an das Pferd treffen zu müssen.

Nach dem Stand der Technik wird der Pferderücken bei der Neuanfertigung von Sätteln beispielsweise mit Hilfe eines, einer Fischgräte ähnelnden, Werkzeuges ausgemessen. Dabei sitzen mehrere parallel angeordnete, flexible Metallstäbe im Winkel von 90° auf einem Verbindungsstück. Das Werkzeug wird auf den Pferderücken aufgelegt, so dass das Verbindungsstück in seiner ganzen Länge den Pferderücken berührt. Die abstehenden Metallstäbe werden dann so gebogen, dass sie sich durch Anlegen an die Kontur des Pferderückens anpassen. Somit lässt sich die Form des Pferderückens nachbilden. Beim Anpassen des Sattels dient das fischgrätenartige Werkzeug als Schablone, aus der man eine ungefähre Nachbildung des Pferderückens durch Ausschneiden von Pappe oder Zurechtsägen von Holzplatten herstellen kann.

Nachteilig ist bei der aktuellen Variante die Unabhängigkeit des Messergebnisses von der Position im Raum, dass heißt die Lage des Verbindungsstücks zur Horizontalen wird nicht erfasst. Legt man das fischgrätenförmige Werkzeug auf den Pferderücken auf, so muss es, anatomisch bedingt nicht unbedingt waagerecht liegen. Hält man hinterher das Werkzeug in der Hand, kann man es beliebig kippen. Das Ergebnis, das in dem Werkzeug abgebildet ist, lässt also keinerlei Rückschlüsse auf die Lage des Sattels zur Horizontalen zu und somit ist eine anatomisch genaue Nachbildung nahezu unmöglich.

Das Dokument US 2003/0221328 A1 beschreibt eine Vorrichtung zur dreidimensionalen Abbildung von Pferderücken, welche ein Untergestell bestehend aus säulenartigen Segmenten mit starren seitlichen Flügeln am oberen Ende aufweist, wobei die Flügel jeweils anhand zweier Streben in ihrer Neigung verstellbar sind. Auf den Flügeln ist zu beiden Seiten des Rückgrats je ein gemeinsames flexibles Querelement in Form eines Blattes befestigt, dessen Krümmung anhand der Flügel auf die gewünschte Form eingestellt werden kann.

Die Aufgabe der Erfindung besteht darin, eine Vorrichtung zu schaffen, mit der der Pferderücken mit ausreichender Genauigkeit und jederzeit reproduzierbar dreidimensional dargestellt werden kann.

Die Aufgabe wird durch die Merkmale des unabhängigen Patentanspruchs gelöst. Weiterbildungen sind in den abhängigen Patentansprüchen angegeben.

Die Vorrichtung zur dreidimensionalen Abbildung von Pferderücken weist mehrere vertikal teleskopierbare und säulenartige Segmente auf, die mit einem Rahmen verbunden sind. Am oberen Ende der Segmente sind regenschirmartig gebogene und in ihrer Krümmung flexible Querelemente vorgesehen, wobei die vertikalen Segmente im Rahmen zueinander variabel beabstandbar und temporär fixierbar ausgebildet sind. Die Krümmung der regenschirmartig gebogenen Querelemente ist gleichfalls einstellbar und temporär fixierbar. Die Querelemente weisen Messpunkte auf, die über Mittel zur Einstellung der Krümmung der Querelemente in einer senkrechten Ebene über die Einstellung der Krümmung der Querelemente variabel anordenbar sind, wobei die Messpunkte auf den Querelementen in der Fläche der dreidimensionalen Kontur des Pferderückens liegen.

Die Vorrichtung wird dahingehend weitergebildet, dass die Mittel zur Einstellung der Krümmung der Querelemente und den darauf angeordneten Messpunkten als teleskopierbare Streben ausgebildet sind, über die die Einstellung der Krümmung der Querelemente und damit die Fixierung der Messpunkte in der senkrechten Ebene der Vorderansicht erfolgt.

Alternativ dazu und besonders vorteilhaft sind die Streben paarweise in der horizontalen Lage und über die Veränderung des Winkels zur Vertikalen mit einem Gleit- und Rastmechanismus einstellbar, so dass auf diese Weise die Messpunkte am Ende der Streben, welche auch als Abstandspunkte bezeichnet werden, exakt in der senkrechten Ebene positioniert werden können.

Ein besonderer Vorteil dieser Alternative besteht darin, dass die beiden Streben zweier zugeordneter Abstandspunkte über den genannten Mechanismus miteinander verbunden und gleichzeitig mit einer Bedienhandlung einstellbar sind. Dies ist in der Praxis möglich, da in aller Regel die zugehörigen Abstandspunkte symmetrisch zur Mittellinie sind. Durch die Gleiteinstellung der Höhe und der Winkel der beiden zugehörigen Streben ist der Einstellprozess neben der Einstellung von Hand auch mit elektrischen Schrittmotoren vorteilhaft möglich.

Eine weitere etwas aufwändigere Ausgestaltung der Streben besteht darin, dass diese teleskopierbar ausgeführt sind und auf einer Seite mit dem Querelement und auf der anderen Seite mit dem säulenartigen Segment verbunden sind, wodurch jede Strebe separat einstellbar ist. Dies ist in jenen besonderen Anwendungsfällen günstig, wenn die einander zugehörigen Abstandspunkte nicht symmetrisch zur Mittellinie sind.

Besonders vorteilhaft und fein einstellbar wird die Vorrichtung dahingehend ausgestaltet, dass die Streben unmittelbar an den Messpunkten mit dem Querelement verbunden sind.

Eine vorteilhafte Ausgestaltung ist darin zu sehen, dass jedem der Messpunkte mit Ausnahme des Messpunktes der Pferderückenmitte eine Strebe zugeordnet ist, wodurch diese Messpunkte besonders exakt eingestellt werden können.

In der Praxis hat sich als eine vorteilhafte Ausgestaltung der Erfindung erwiesen, dass sechs Messpunkte neben dem Messpunkt der Pferderückenmitte auf einem Querelement angeordnet sind, wobei die sechs Messpunkte als Messpunktpaare ausgebildet sind und die Punkte eines Paares jeweils in einer horizontalen Ebene liegen.

Weiterhin als zweckmäßig hat sich erwiesen, dass die Vorrichtung fünf vertikale säulenartige teleskopierbare Segmente aufweist, wobei jedes Segment am Querelement drei Messpunktpaare und den Messpunkt der Pferderückenmitte aufweist.

Zur Aufnahme der Messwerte am Pferderücken für die Einstellung der Messpunkte am Pferderückenabbilder ist ein Rückenlineal vorgesehen, welches nicht Bestandteil der vorliegenden Erfindung bildet. Das Rückenlineal ist ein starres Profil mit einem endseitigen Auflagepunkt A und eine Lagemesseinrichtung für die horizontale Lage des Profils. Weiterhin sind Mittel zur Höhenverstellung vorgesehen, um die horizontale Lage des Profils einstellen zu können.

Das Rückenlineal ist in einer bevorzugten Ausgestaltung dadurch gekennzeichnet, dass die Lagemesseinrichtung für die horizontale Lage des Profils als Libelle ausgestaltet ist. Unter einer Libelle wird ein mit einer Flüssigkeit und einer Gasblase gefüllter durchsichtiger Hohlkörper zur Überprüfung der horizontalen oder vertikalen Lage des Profils verstanden. Das Mittel zur Höhenverstellung ist als verstellbarer Gewindestab ausgebildet. Alternativ ist das Mittel zur Höhenverstellung als Distanzstab oder als Teleskopstab ausgebildet, wobei es darauf ankommt, dass eine schnelle und sichere Lageanpassung des Rückenlineals in der horizontalen Lage möglich ist.

Das Verfahren zur dreidimensionalen Abbildung von Pferderrücken mittels der erfindungsgemäßen Vorrichtung zur dreidimensionalen Abbildung von Pferderrücken sowie einem Rückenlineal und flexiblen Biegelinealen besteht aus folgenden Verfahrensschritten:
Ausmessen des Pferderückens und Ermittlung der Messwerte zu den Messpunkten der dreidimensionalen Kontur des Pferderückens mit dem horizontal justierten Rückenlineal und den flexiblen Biegelinealen sowie Protokollierung der Messwerte,
Übertragung der Messwerte auf die Vorrichtung zur dreidimensionalen Abbildung von Pferderücken und Einstellung der Messpunkte.

Unter Messwerten sind die Abstände von zwei Punkten zu verstehen, wohingegen Messpunkte im Sinne der Erfindung, Punkte im Raum sind, die sich aus den über die Messwerte ermittelten Koordinaten ergeben.

Mit der Vorrichtung zur Nachbildung eines zu vermessenden Pferderückens kann die Kontur eines Pferderückens naturgetreu, maßstabsgerecht und dreidimensional richtig im Raum wiedergegeben werden. Damit kann der Reitsattel, der das Bindeglied zweier verschiedener Anatomien, der des Menschen und der des Pferdes, darstellt, in seiner Lage auf dem Pferderücken den konkreten Erfordernissen des einzelnen Pferdes entsprechend gestaltet werden.

Die Vermessung des Pferderückens, mit der die Werte für die Anwendung der erfindungsgemäßen Vorrichtung, auch HBST für Horse Back Simulation Tool genannt, gewonnen werden, erfolgt mittels eines Rückenlineals, das aus einem starren Profil, bevorzugt aus Metall, mit integrierter gefasster Libelle und einem in der Höhe verstellbaren Gewindestab zur Lagejustierung des Rückenlineals, der senkrecht angebracht ist, besteht.

Im vorgeschenen Gebrauch setzt das Werkzeug vorn auf dem höchsten Dornfortsatz des Pferderückens auf und hinten berührt der Gewindestab den Pferderücken.

Durch Verstellen des Gewindestabs in vertikaler Richtung wird das Rückenlineal mit Hilfe der Libelle in eine genau horizontale Lage gebracht. Es versteht sich, die horizontale Justierung des Rückenlineals auch mit Hilfe eines Gyroskops oder ähnlicher Justiereinrichtungen sowie teleskopierbare Elemente zur Lageanpassung an Stelle des Gewindestabes erfolgen kann.

Auf der Mitte des Pferderückens werden charakteristische Punkte markiert. An diesen Punkten werden die Höhen vom Pferderücken bis zum horizontalen Metallprofil gemessen.

Weiterhin werden die Abstände dieser charakteristischen Punkte voneinander ermittelt.

Mit an diesen Punkten vertikal an den sogenannten Schnittlinien angelegten flexiblen Biegelinealen wird die Kontur Pferderücken nachgebildet.

Auf Grund dieser ermittelten Daten, die schriftlich in einer Matrix erfasst werden, kann nun das HBST eingestellt werden.

Konzeptionsgemäß besteht das HBST beispielsweise aus fünf Ständern, die auf einem Gestell verschiebbar befestigt und durch eine Führung in horizontaler Richtung einstellbar sind. Jeder dieser Ständer besteht wiederum aus einem Kopfteil, das in der Vertikalen teleskopartig verstellbar ist. Am oberen Ende des Kopfteils befindet sich ein flexibles Federstahlband als Querelement, das über einstellbare Streben verstellt werden kann. Alle verstellbaren Glieder der Vorrichtung sind an jeder gewählten Position fixierbar ausgeführt.

Auf diese Weise kann der Pferderücken naturgetreu und anatomisch korrekt nachgebildet werden. Der Vorteil besteht darin, dass ein anzupassender Sattel direkt auf den "nachgebildeten künstlichen Pferderücken" aufgelegt werden kann. Die durch das HBST gebildete Form kann vom Sattelhersteller, Sattelanpasser, oder Reitsporthändler als Schablone verwenden und ist dadurch in die Lage versetzt, den Sattel für das vermessene Pferd passend herzustellen, anzupassen oder aus einer Anzahl von Ferig-Sätteln den passenden herauszufiltern.

Die Erfindung weist eine Reihe von Vorteilen gegenüber dem Stand der Technik auf.

Es besteht ein eindeutiger technischer Vorteil darin, dass Winkelfehler ausgeschlossen werden können und die Anpassung des Sattels wesentlich genauer wird. Da mit dieser Vorrichtung und dem Verfahren zu seiner Benutzung die Wahrscheinlichkeit einer aufwendigen Nachbesserung minimiert wird, ergibt sich ein ökonomischer Vorteil. Mittels der dreidimensionalen Verstellbarkeit des HBST können verschiedenste Pferderückenformen in kürzester Zeit ohne Materialverbrauch dargestellt werden. Ein Sattelhersteller, Sattelanpasser oder Reitsporthändler wird dadurch in die Lage versetzt, auch mit räumlich weit entfernt gewonnenen Daten eine genaue Darstellung der Beschaffenheit eines Pferderückens zu erhalten und dafür einen Sattel nach diesen Maßen herzustellen oder zu bearbeiten.

Weitere Einzelheiten, Merkmale und Vorteile von Ausgestaltungen der Erfindung ergeben sich aus der nachfolgenden Beschreibung von Ausführungsbeispielen mit Bezugnahme auf die zugehörigen Zeichnungen. Es zeigen:
- Fig. 1:: Werkzeug zur Gewinnung von Messpunkten
- Fig. 2:: Matrix zur Erfassung der Messergebnisse
- Fig. 3a:: flexibles Biegelineal in der Draufsicht

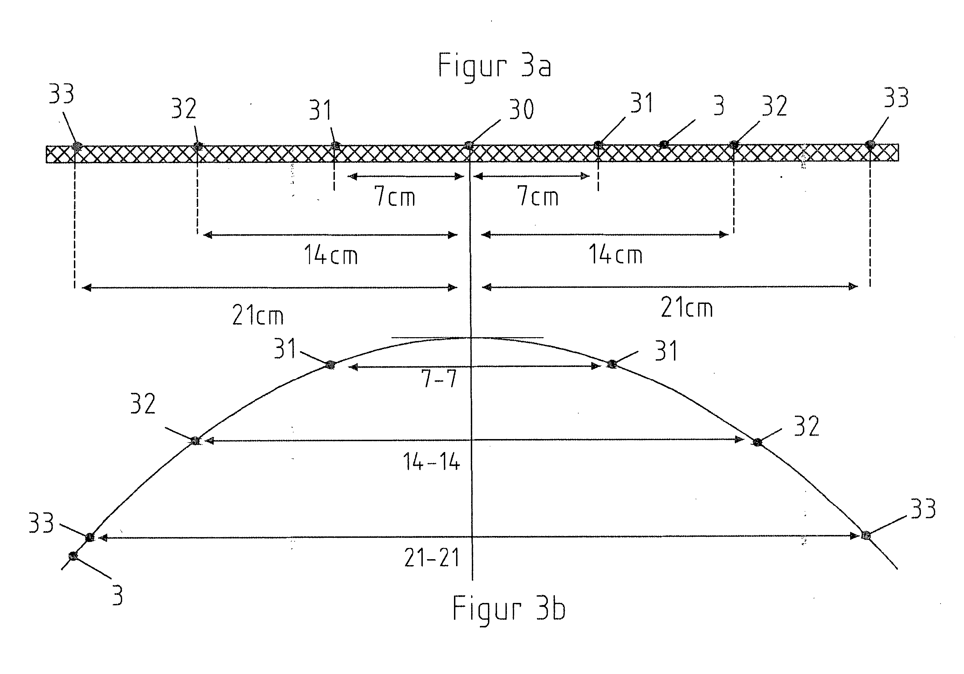

- Fig. 3a:: flexibles Biegelineal in der Seitenansicht

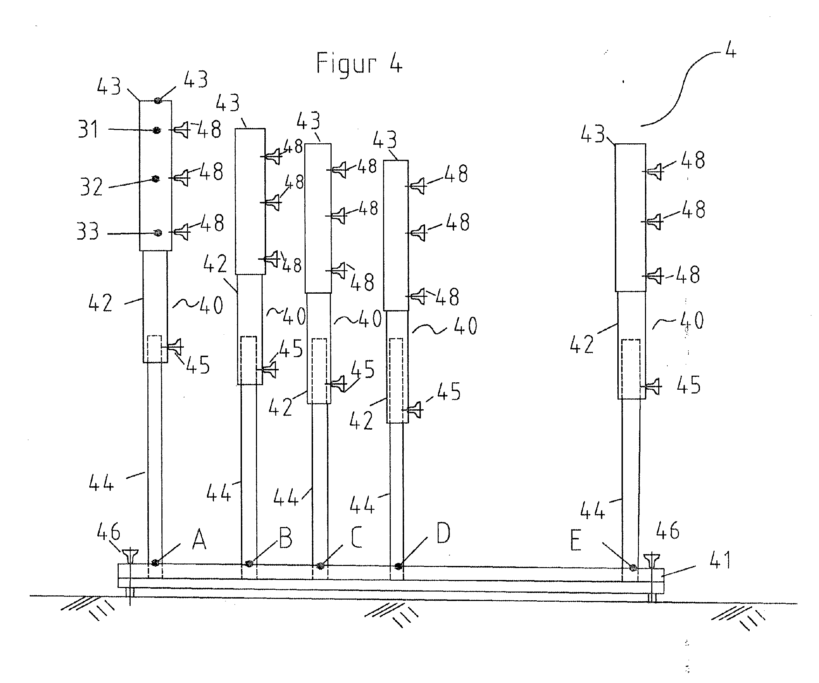

- Fig. 4:: Seitenansicht des Pferderückenabbilders

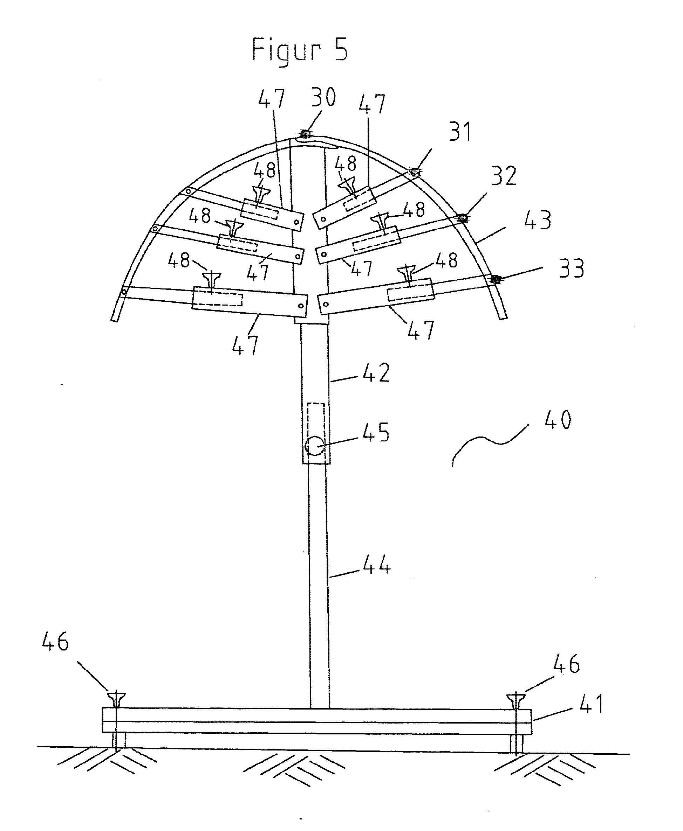

- Fig. 5:: Vorderansicht des Pferderückenabbilders

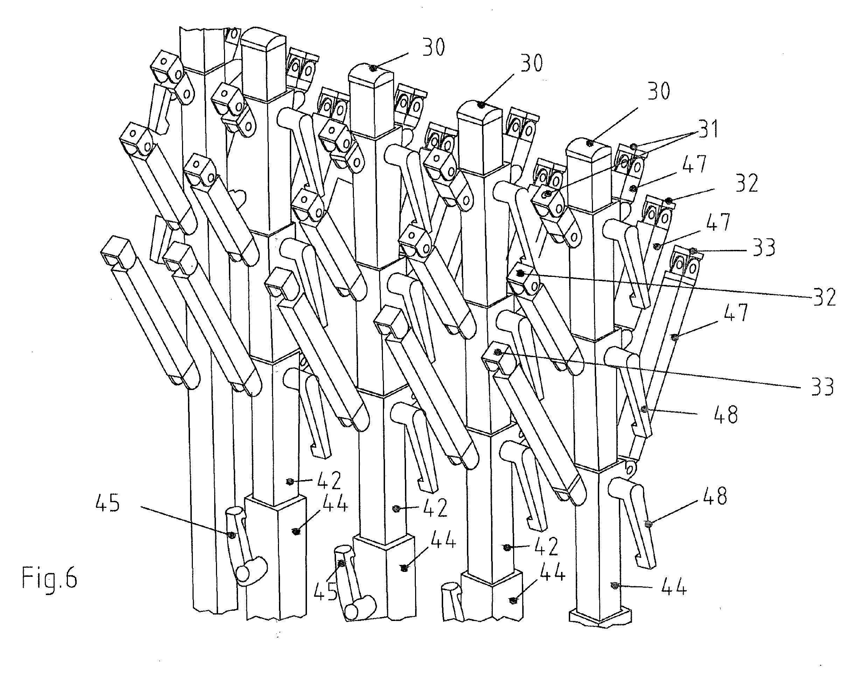

- Fig. 6:: Segmentkopfteile mit Streben

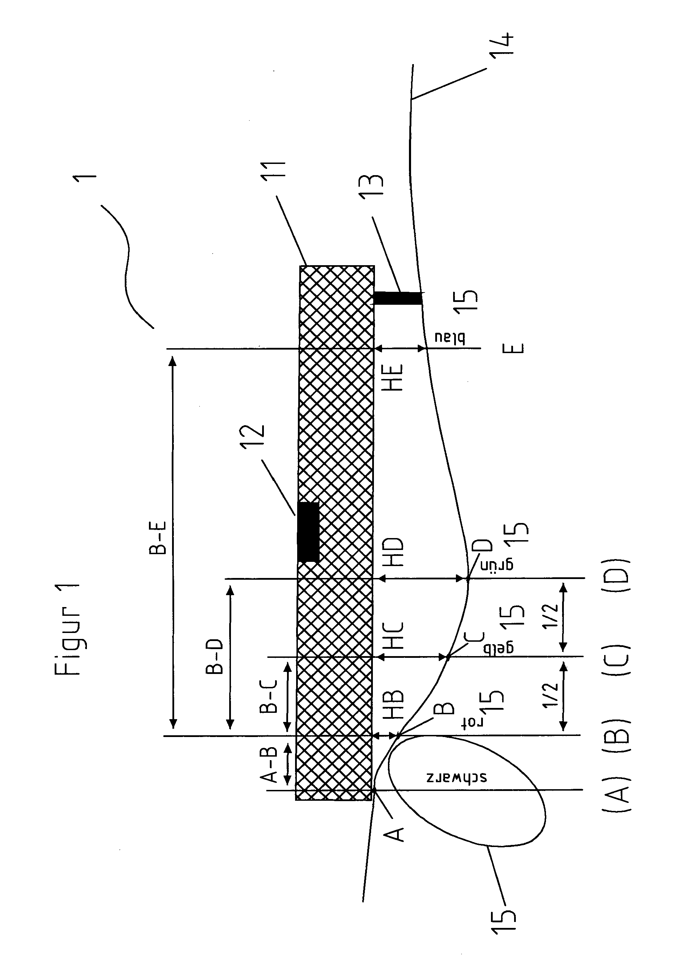

Die Figur 1 zeigt das auf die Pferderückenkonturlinie 14 aufgelegte Werkzeug 1 zur Gewinnung von Messpunkten, welches auch als Rückenlineal bezeichnet wird, das aus dem starren Metallprofil 11 mit integrierter Libelle 12 und einem senkrecht nach unten angeordneten verstellbaren Gewindestab 13 zur horizontalen Lagejustierung besteht. A bezeichnet den Auflagepunkt des Werkzeugs 1 auf der Pferdeschulter am Punkt ihrer stärksten Wölbung, B bildet die Basislinie am Pferdeschulteransatz, C bezeichnet die Trapezlinie als Mitte zwischen B und D, D kennzeichnet den tiefsten Punkt Pferderückens und E markiert die Linie abwärts vom 18. Brustwirbel Pferderückens.

Die Libelle 12 dient der genauen horizontalen Ausrichtung des starren Metallprofils 11 mit Hilfe verstellbaren Gewindestabs 13.

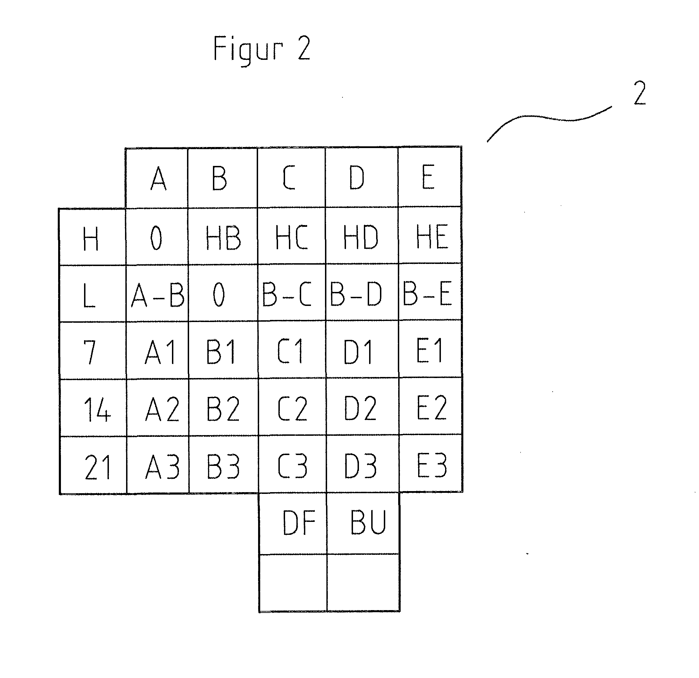

Figur 2 zeigt die Matrix 2 zur übersichtlichen Erfassung der mit Hilfe des Rückenlineals erfassten Messwerte, die für die Einstellung der Vorrichtung des Pferderückenabbilders HBST verwendet werden.

Figur 3a zeigt ein flexibles Biegelineal 3 in der Draufsicht und Fig. 3b zeigt ein flexibles Biegelineal 3 in der Seitenansicht. Nach beiden Seiten von der Pferderückenmitte 30 aus sind Messpunkte vorgesehen, auch als Abstandspunkte bezeichnet, bei 7 cm mit dem Bezugszeichen 31 das Maß 7-7 bildend, an den Abstandspunkten bei 14 cm mit dem Bezugszeichen 32 das Maß 14-14 bildend und an den Abstandspunkten bei 21 cm mit dem Bezugszeichen 33, das Maß 21-21 bildend.

Figur 4 stellt die Vorrichtung 4 zum Abbilden eines Pferderückens HBST in einer Seitenansicht dar. Ein Rahmen 41 mit Führungen für verschiebbare Segmente 40 ist mit Justierschrauben 46 versehen, die das Einstellen einer horizontalen Lage des Rahmens 41 ermöglichen. Mehrere Segmente 40 sind verschiebbar auf dem Rahmen 41 angeordnet und bestehen aus einem Ständerfuß 44, einem darauf teleskopartig vertikal verstellbaren aufgesetzten Segmentkopfteil 42, das an seiner oberen Begrenzung ein Querelement als Federstahlband 43 trägt. Die Höhenverstellung jedes Segmentkopfteils 42 wird mittels Feststellschrauben 45 oder durch eine Gewindespindel fixiert. Weiterhin dargestellt sind die Feststellschrauben 48 für die in dieser Figur nicht dargestellten teleskopierbaren Streben 47. In der Seitenansicht ist jeweils nur eine Seite der Querelemente 43 mit den Messpunkten 31, 32 und 33 sowie dem Messpunkt 30 der Pferderückenmitte beispielhaft am linken Querelement 43 dargestellt.

Figur 5 zeigt die Vorderansicht der Vorrichtung zum Abbilden eines Pferderückens, in der der Aufbau eines Segmentes 40 vorteilhaft darstellbar ist. Die Messpunkte 30, 31, 32 und 33 liegen in einer in der Vorderansicht dargestellten senkrechten Ebene. Der Grundaufbau der Vorrichtung besteht aus dem Rahmen 41 und dem säulenartigen Segment 40 sowie dem Querelement 43 und ist bereits in der Beschreibung zu Figur 4 angegeben.

Das höhenverstellbare Segmentkopfteil 42 trägt mehrere einzeln verstellbare Streben 47, die mit ihrer eingestellten Länge die Krümmung und damit die Form des Federstahlbandes 43 bestimmen. Durch die an den verstellbaren Streben 47 angebrachten Feststellschrauben 48 kann die jeweilig zugehörige Länge der Strebe 47 und damit die Form des Querelementes 43, ausgeführt als Federstahlband, fixiert werden.

Auf dem Querelement 43 sind die Messpunkte 30, 31, 32 und 33 angeordnet, wobei die Querelemente 43 die Messpunkte mit dem Segmentkopfteil 42 verbinden, wodurch die im Querschnitt regenschirmartige Kontur entsteht.

In Figur 6 ist eine bevorzugte Ausführungsform der im Ständerfuß 44 geführten und über Arretierungsmittel 45 in der Höhe positionierbare Segmentkopfteile 42 mit den Streben 47 dargestellt. Im Unterschied zu der in Figur 5 dargestellten Ausgestaltung sind die Streben 47 für zwei zugehörige Abstandspunkte auf gleichem horizontalen Niveau über einen Gleit- und Rastmechanismus miteinander derart verbunden, dass beide Streben 47 in vertikaler Richtung am Segmentkopfteil 42 gleitend in ihrer Höhe und im Anstellwinkel zur Vertikalen verstellbar sind. Die Streben 47 haben dabei eine konstante und nicht teleskopierbare Länge und die Messpunkte 31, 32 und 33 am Ende der Streben 47 werden über die gemeinsame Bewegung des Mechanismus mit den Streben 47 in vertikaler Richtung und die damit verbundene Drehbewegung der Streben 47 mit einer Veränderung des Anstellwinkels platziert. Dabei wird davon ausgegangen, dass der vermessene Pferderücken in Bezug auf die Mittellinie eine symmetrische Kontur hat.

Von besonderem Vorteil dieser Ausgestaltung ist, dass mit nur einer Stellbewegung am Arretierungsmittel 48 des Gleit- und Rastmechanismus beide Streben 47 in Ihrer Höhe und in ihrer Neigung eingestellt werden können und somit ein Messpunktpaar 31, 32 und 33 gleichzeitig in der senkrechten Ebene der Vorderansicht positioniert werden kann.

Die translatorische Bewegung der paarweise verbundenen Streben 47 und deren davon abhängige Winkeleinstellung sowie die translatorische Bewegung des gesamten Segmentkopfteils 42 ermöglicht den Einsatz von Schrittmotoren für die Einstellung der Messpunkte 30, 31, 32 und 33. Damit ist eine elektrische und sogar vollautomatische Einstellung der Messpunkte möglich.

### LISTE DER BEZUGSZEICHEN

- 1: Rückenlineal, Werkzeug zur Gewinnung von Messwerten
- 11: starres Profil
- 12: Lagemesseinrichtung, Libelle
- 13: Mittel zur Höhenverstellung, Gewindestab, Distanzstab, Teleskopstab
- 14: Pferderückenkonturlinie
- A: Auflagepunkt auf der Pferdeschulter an ihrer stärksten Wölbung
- B: Basislinie am Pferdeschulteransatz
- C: Trapezlinie als Mitte zwischen B und D
- D: Tiefster Punkt /Deep Point) Pferderückens
- E: Linie zum 18. Brustwirbel Pferderückens
- HB - HE: Höhen an den Linien B bis E
- A-B: Abstand zwischen A und B
- B-C: Abstand zwischen B und C
- B-D: Abstand zwischen B und D
- B-E: Abstand zwischen B und E
- 15: Farbkennzeichnung der Scheitelpunkte der Schnittlinien A bis E
- 2: Matrix zur Erfassung der Messwerte
- 3: flexibles Biegelineal
- 30: Messpunkt Pferderückenmitte
- 31: Messpunkt erster Abstandspunkt 7 cm von der Pferderückenmitte 30,
- 32: Messpunkt zweiter Abstandspunkt 14 cm von der Pferderückenmitte 30,
- 33: Messpunkt dritter Abstandspunkt 21 cm von der Pferderückenmitte 30,
- 4: Vorrichtung zum Abbilden eines Pferderückens (HBST)
- 40: teleskopierbares säulenartiges Segment
- 41: Rahmen mit Führung für die Segmente 40
- 42: Höhenverstellbares Segmentkopfteil
- 43: Querelement Segmentkopfteils, Federstahlband
- 44: Ständerfuß
- 45: Arretierungsmittel für die Höhenverstellung des Segmentkopfteils 42, Feststellschraube
- 46: Justierschraube für den Rahmen 41
- 47: Mittel zur Einstellung der Krümmung der Querelemente, Streben
- 48: Arretierungsmittel für Streben 47, Feststellschraube

## Patentansprüche

1. Vorrichtung (4) zur dreidimensionalen Abbildung von Pferderücken mittels Messpunkten (30, 31, 32, 33), aufweisend mehrere vertikal teleskopierbare, säulenartige und in einem Rahmen (41) zueinander variabel beabstandbar und temporär fixierbare Segmente (40), wobei jedes Segment (40) an seinem oberen Ende ein regenschirmartig gebogenes und in seiner Krümmung flexibles Querelement (43) aufweist und wobei die Krümmung jedes Querelementes (43) mittels mehrerer verstellbarer Streben (47) einstellbar und temporär fixierbar ist, wobei die verstellbaren Streben (47) auf einer Seite mit dem Querelement (43) und auf der anderen Seite mit einem Segmentkopfteil (42) oder einem Gleit- und Rastmechanismus verbunden sind und wobei jedes Querelement (43) mehrere Messpunkte (30, 31, 32, 33) aufweist, die über zugeordnete verstellbare Streben (47) zur Einstellung der Krümmung der Querelemente (43) in einer senkrechten Ebene variabel anordenbar ausgebildet sind, wobei die Messpunkte (30, 31, 32, 33) der Querelemente (43) so angeordnet werden können, dass sie in der Fläche der dreidimensionalen Kontur des Pferderückens liegen.

2. Vorrichtung (4) nach Anspruch 1, **dadurch gekennzeichnet, dass** die verstellbaren Streben (47) zur Einstellung der Krümmung der Querelemente (43) als Gleit- und Rastmechanismus ausgebildet sind, wobei jeweils zwei Streben (47) gleicher Länge zur Darstellung eines Messpunktpaares (31, 32, 33) derart miteinander verbunden sind, dass über die horizontale Lage auch die Neigung der Streben (47) gegenüber der Vertikalen einstellbar und fixierbar ist.

3. Vorrichtung (4) nach Anspruch 1, **dadurch gekennzeichnet, dass** die verstellbaren Streben (47) zur Einstellung der Krümmung der Querelemente (43) als teleskopierbare Streben (47) ausgebildet sind, über die jeweils einzeln die Einstellung der Krümmung der Querelemente (43) und damit die Fixierung der Messpunkte (31, 32, 33) in der senkrechten Ebene erfolgt.

4. Vorrichtung (4) nach einem der Ansprüche 1 bis 3, **dadurch gekennzeichnet, dass** die Streben (47) an den Messpunkten (31, 32, 33) mit den Querelementen (43) verbunden sind.

5. Vorrichtung (4) nach einem der Ansprüche 1 bis 4, **dadurch gekennzeichnet, dass** jedem der Messpunkte (31, 32, 33) eine Strebe (47) zugeordnet ist.

6. Vorrichtung (4) nach Anspruch 5, **dadurch gekennzeichnet, dass** sechs Messpunkte (31, 32, 33) neben dem Messpunkt der Pferderückenmitte (30) auf einem Querelement (43) angeordnet sind.

7. Vorrichtung (4) nach einem der Ansprüche 1 bis 6, **dadurch gekennzeichnet, dass** die Vorrichtung (4) mindestens fünf Segmente (40) aufweist.

## Claims

1. Device (4) for three-dimensional imaging of horsebacks by means of measuring points (30, 31, 32, 33) comprising a plurality of vertically telescopic, columnar segments (40) which can be spaced apart from each other variably and which can be fixed temporarily within a frame (41), wherein each segment (40) at its upper end has a cross member (43) being bent in an umbrella-like manner and having a flexible curvature, wherein the curvature of each cross member (43) is adjustable and temporarily fixable by means of a plurality of adjustable struts (47), wherein said adjustable struts (47) on one side are connected to the cross member (43) and on the other side are connected to a segment header (42) or are connected to a sliding and latching mechanism, and wherein each cross member (43) has several measuring points (30, 31, 32, 33) which are configured such that they can be arranged variably in a vertical plane by means of assigned adjustable struts (47) for adjustment of the curvature of the cross members (43), wherein the measuring points (30, 31, 32, 33) of the cross members (43) can be arranged such that they lie in the area of the three-dimensional contour of the horseback.

2. Device (4) according to claim 1,
**characterized in that** the adjustable struts (47) are configured as a sliding and latching mechanism for adjustment of the curvature of the cross members (43), wherein two struts (47) of equal length are each interconnected for representation of a measure point pair (31, 32, 33) in such a manner that the inclination of the struts (43) with respect to the vertical can equally be adjusted and fixed via the horizontal position.

3. Device (4) according to claim 1,
**characterized in that** the adjustable struts (47) are configured as telescopic struts (47) for adjustment of the curvature of the cross members (43) via which the adjustment of the curvature of the cross members (43) and thus the fixation of the measuring points (31, 32, 33) takes place individually in the vertical plane.

4. Device (4) according to any of claims 1 to 3,
**characterized in that** the struts (47) are connected to the cross members (43) at the measuring points (31, 32, 33).

5. Device (4) according to any of claims 1 to 4,
**characterized in that** one strut (47) is assigned to each of the measuring points (31, 32, 33).

6. Device (4) according to claim 5,
**characterized in that** six measuring points (31, 32, 33) are disposed adjacent to the measuring point of the middle of the horseback (30) on a cross member (43).

7. Device (4) according to any of claims 1 to 6,
**characterized in that** the device (4) has at least five segments (40).

## Revendications

1. Dispositif de représentation tridimensionnelle de dos de chevaux au moyen de points de mesure (30, 31, 32, 33) présentant une pluralité de segments (40) étant verticalement télescopique, ayant une forme colonnaire et pouvant être espacés l'un de l'autre de façon variable et pouvant être fixés temporairement dans un cadre (41), dans lequel chaque segment (40) à son extrémité supérieure présente une traverse (43) étant courbée en forme de parapluie et ayant une courbure flexible, et dans lequel la courbure de chaque traverse (43) est réglable et temporairement fixable au moyen d'une pluralité d'entretoises (47) réglables, dans lequel les entretoises (47) réglables sur un côté sont reliées à la traverse (43) et sur l'autre côté sont reliées à une têtière du segment (42) ou sont reliées à un mécanisme de coulissement et de verrouillage, et dans lequel chaque traverse (43) présente une pluralité de points de mesure (30, 31, 32, 33) étant conçus de telle manière que ceux-ci peuvent être agencés dans un plan vertical de manière variable au moyen des entretoises (47) réglables attribués pour le réglage de la courbure des traverses (43), dans lequel les points de mesure (30, 31, 32, 33) des traverses (43) peuvent être agencés de telle manière que ceux-ci se trouvent dans la zone de la contour tridimensionnelle au milieu du dos de cheval.

2. Dispositif (4) selon la revendication 1,
**caractérisé en ce que** les entretoises (47) réglables pour le réglage de la courbure des traverses (43) sont conçues en tant qu'un mécanisme de coulissement et de verrouillage, dans lequel deux entretoises (47) de longueur égale sont respectivement reliées l'une à l'autre pour la représentation d'une paire de points de mesure (31, 32, 33) de telle manière que l'inclinaison des entretoises (47) par rapport à la verticale est également réglable et fixable par la position horizontale.

3. Dispositif (4) selon la revendication 1,
**caractérisé en ce que** les entretoises (47) réglables pour le réglage de la courbure des traverses (43) sont conçues en tant qu'entretoises (47) télescopiques permettant que le réglage de la courbure des traverses (43) et par conséquent la fixation des points de mesure (31, 32, 33) soient effectués individuellement dans le plan vertical.

4. Dispositif (4) selon l'une quelconque des revendications 1 à 3,
**caractérisé en ce que** les entretoises (47) au niveau des points de mesure (31, 32, 33) sont reliées aux traverses (43).

5. Dispositif (4) selon l'une quelconque des revendications 1 à 4,
**caractérisé en ce qu'**une entretoise est attribuée à chaque point de mesure (31, 32, 33).

6. Dispositif (4) selon la revendication 5,
**caractérisé en ce que** six points de mesure (31, 32, 33) adjacents du point de mesure au milieu du dos de cheval (30) sont disposés sur une traverse (43).

7. Dispositif selon l'une quelconque des revendications 1 à 6,
**caractérisé en ce que** ledit dispositif (4) présente au moins cinq segments (40).
